Europäisches Patentamt

European Patent Office

Office européen des brevets

(19)

(11) Veröffentlichungsnummer: **0 011 089**
**B1**

(12) # EUROPÄISCHE PATENTSCHRIFT

(45) Veröffentlichungstag der Patentschrift:
15.07.81

(21) Anmeldenummer: **79102927.5**

(22) Anmeldetag: **13.08.79**

(51) Int. Cl.³: **C 07 C 143/55**, C 07 B 11/00 //
C07C143/60

(54) Verfahren zur Herstellung von Nitro-T-Säure (8-Nitronaphthalin-1,3,6-trisulfonsäure).

(30) Priorität: **28.08.78 DE 2837498**

(43) Veröffentlichungstag der Anmeldung:
**28.05.80 Patentblatt 80/11**

(45) Bekanntmachung des Hinweises auf die Patenterteilung:
**15.07.81 Patentblatt 81/28**

(84) Benannte Vertragsstaaten:
**BE CH DE FR GB IT**

(56) Entgegenhaltungen:
**DE-C-56 058**

(73) Patentinhaber: **BAYER AG, Zentralbereich Patente,
Marken und Lizenzen, D-5090 Leverkusen 1, Bayerwerk
(DE)**

(72) Erfinder: **Kaiser, Reinhard, Dr., Morgengraben 14,
D-5000 Köln 80 (DE)**
Erfinder: **Behre, Horst, Dr., Im Hellsiefen 4,
D-5068 Odenthal (DE)**
Erfinder: **Dammann, Jürgen, Dr., Wolfskaul 5,
D-5000 Koeln 80 (DE)**
Erfinder: **Pütter, Rolf, Dr., Poststrasse 7,
D-4000 Düsseldorf 1 (DE)**
Erfinder: **Vogel, Axel, Dr., In der Hildscheid 5,
D-5068 Odenthal 1 (DE)**

ACTORUM AG.

## Verfahren zur Herstellung von Nitro-T-Säure (8-Nitronaphthalin-1,3,6-trisulfonsäure)

Die vorliegende Erfindung betrifft ein Verfahren zur Herstellung von Nitro-T-Säure durch Nitrierung von Naphthalin-1,3,6-trisulfonsäure.

Es ist bekannt, Nitro-T-Säure durch Nitrieren des bei der Naphthalintrisulfierung mit Schwefelsäure und Oleum anfallenden Isomerengemisches von Naphthalintrisulfonsäuren, welches als Hauptbestandteil Naphthalin-1,3,6-trisulfonsäure enthält, in Schwefelsäure als Lösungsmittel mit Salpetersäure herzustellen. (s. DRP 56058 und F.I.A.T., Final Report 1016, Seiten 32 bis 39). Bei den aus der Literatur bekannten Herstellungsverfahren arbeitet man so, dass man die bei der Naphthalintrisulfierung erhaltene 150 bis 155°C heisse Lösung der Naphthalintrisulfonsäuren in Schwefelsäure auf 100°C abkühlt, mit Wasser verdünnt und weiter auf 80°C abkühlt.

Dann überführt man den Ansatz in den Nitrierkessel, kühlt dort auf 35 bis 40°C ab und nitriert im Verlauf von ca. 10 Stunden mit 86%iger Salpetersäure (12 % Schwefelsäure, 2% Wasser) bei 35 bis 40°C. Die Reaktionswärme wird über die Kesselwand durch Kühlung mit Kühlwasser abgeführt.

Es wurde nun ein Verfahren zur Herstellung von 8-Nitronaphthalin-1,3,6-trisulfonsäure aus Naphthalin-1,3,6-trisulfonsäure oder diese enthaltenden Gemischen durch Nitrierung mit Salpetersäure in Schwefelsäure gefunden, das dadurch gekennzeichnet ist, dass man zu mindestens 80% ausreagiertes Nitriergemisch vorlegt, Naphthalin-1,3,6-trisulfonsäure oder diese enthaltende Gemische gelöst in Schwefelsäure zufügt, gleichzeitig 1,0 bis 1,4 Mol Salpetersäure pro Mol Naphthalin (in Form von Naphthalinsulfonsäure vorliegend) und gegebenenfalls Wasser in der Weise zufügt, dass man eine Schwefelsäurekonzentration im Bereich 86 bis 94 Gew.-% (Schwefelsäure, bezogen auf die Summe aus allem vorhandenem Wasser und Schwefelsäure) während der Nitrierung einhält, das Reaktionsgemisch gut durchmischt und die Reaktionstemperatur im Bereich 30 bis 60°C hält.

In das erfindungsgemässe Verfahren kann man reine Naphthalin-1,3,6-trisulfonsäure, gelöst in Schwefelsäure, einsetzen. Man kann auch Naphthalin-1,3,6-trisulfonsäure enthaltende Gemische, gelöst in Schwefelsäure, einsetzen. Vorzugsweise werden Naphthalin-1,3,6-trisulfonsäure enthaltende Gemische, gelöst in Schwefelsäure, eingesetzt, wie sie bei der Trisulfierung von Naphthalin anfallen. Beispielsweise kann man das Gemisch einsetzen, das bei der Trisulfierung von Naphthalin gemäss F.I.A.T., Final Report, a.a.O. anfällt. Das auf diese Weise hergestellte Gemisch enthält in ca. 100%iger Schwefelsäure ca. 40 bis 48% Gew.-% Naphthalintrisulfonsäuren, von denen etwa 75 Gew.-% Naphthalin-1,3,6-trisulfonsäure, etwa 8 Gew.-% Naphthalin-1,3,5-trisulfonsäure und etwa 12 Gew.-% Naphthalin-1,3,7-trisulfonsäure sind. Neben Schwefelsäure und Naphthalintrisulfonsäuren kann das Gemisch noch in geringen Mengen andere Naphthalinsulfierungsprodukte und/oder Oxidationsprodukte des Naphthalins enthalten. Man kann in das erfindungsgemässe Verfahren auch auf andere Weise hergestellte Naphthalintrisulfonsäure-Gemische einsetzen.

Da die Löslichkeit der Naphthalintrisulfonsäuren und insbesondere der Naphthalin-1,3,6-trisulfonsäure in 100%iger Schwefelsäure bei Raumtemperatur gering ist, verwendet man zur Eingabe in das erfindungsgemässe Verfahren vorzugsweise Lösungen mit einer Temperatur über 100°C. Sehr gut geeignet sind im allgemeinen die aus der Naphthalintrisulfierung direkt erhältlichen Lösungen, die beispielsweise eine Temperatur im Bereich von 140 bis 160°C aufweisen.

Das vorgelegte Nitriergemisch kann beispielsweise zu mindestens 80%, vorzugsweise zu mindestens 90% ausreagiert sein. Besonders bevorzugt wird ein zu 95 bis 99% ausreagiertes Nitriergemisch vorgelegt. Bei diskontinuierlicher Fahrweise kann ein Teil des vorhergegangenen Ansatzes vorgelegt werden, bei kontinuierlicher Arbeitsweise kann man so arbeiten, dass man Naphthalin-1,3,6-trisulfonsäure und Salpetersäure in einen Schlaufenreaktor (vgl. Ullmann Enzyklopädie der technischen Chemie, Verlag Chemie, Weinheim/Bergstr., Band 1, Seite 227 und Band 4, Seite 9) eingibt, in dem vor der Zugabestelle das Nitriergemisch zu mindestens 80%, vorzugsweise zu mindestens 90%, ganz besonders bevorzugt zu 95 bis 99%, nitriert ist.

Die Menge der Naphthalin-1,3,6-trisulfonsäure enthaltenden Schwefelsäurelösung kann, bezogen auf vorgelegtes ausreagiertes Nitriergemisch, im Falle der diskontinuierlichen Durchführung des erfindungsgemässen Verfahrens beispielsweise 5 bis 5.000-Gew.-%, vorzugsweise 300 bis 1.000 Gew.-% betragen. Im Falle der kontinuierlichen Durchführung des erfindungsgemässen Verfahrens kann in das im Kreis geführte Reaktionsgemisch maximal soviel Naphthalin-1,3,6-trisulfonsäure enthaltende Schwefelsäurelösung eingegeben werden, dass die eingegebene Menge Naphthalin-1,3,6-trisulfonsäure zu mindestens 80%, vorzugsweise zu mindestens 90% im ersten Durchgang nitriert wird.

In das erfindungsgemässe Verfahren werden gleichzeitig mit der Naphthalin-1,3,6-trisulfonsäure enthaltenden Schwefelsäurelösung 1,0 bis 1,4 Mol Salpetersäure pro Mol Naphthalin (in Form von Naphthalinsulfonsäuren vorliegend) zugegeben. Vorzugsweise beträgt diese Salpetersäuremenge 1,1 bis 1,25 Mol. Die Salpetersäure kann beispielsweise in reiner Form (100%ige $HNO_3$) oder in Form von wässriger Salpetersäure mit Gehalten von über 60 Gew.-% $HNO_3$ zugefügt werden. Beispielsweise kann 60 bis 100%ige Salpetersäure eingesetzt werden. Besonders bevorzugt wird wässrige Salpetersäure mit einer Konzentration von 67 bis 68 Gew.-% (sogenannte Azeotropsäure) verwendet.

Es ist ein wesentliches Merkmal des erfindungsgemässen Verfahrens, dass sich die Konzentration der Schwefelsäure während der Nitrierung praktisch nicht oder nur in geringem Masse ändert und im Bereich 86 bis 94% liegt (Schwefelsäure bezogen auf Summe aus Wasser und Schwefelsäure). Vorzugsweise liegt die Schwefelsäurekonzentration im Bereich von 88 bis 92%. Diese Konzentration kann beeinflusst werden durch das während der Reaktion freiwerdende Wasser, durch das gegebenenfalls mit der Salpetersäure eingebrachte Wasser und durch gegebenenfalls separat zugegebenes Wasser. Um die gewünschte Konzentration an Schwefelsäure einzustellen und zu halten ist es deshalb erforderlich, die im Nitriergemisch vorhandene Wassermenge genau zu kontrollieren. Die Einstellung der Schwefelsäurekonzentration kann dadurch erfolgen, dass man zusammen mit der Salpetersäure oder separat davon die notwendige Wassermenge zugibt. Überraschenderweise hat sich gezeigt, dass man in den meisten Fällen eine geeignete Schwefelsäurekonzentration erhält, wenn man die Salpetersäure in Form von wässriger Salpetersäure mit einer Konzentration von 67 bis 68 Gew.-% (sog. Azeotropsäure) in Mengen von 1,1 bis 1,25 Mol pro Mol Naphthalin (in Form von Naphthalinsulfonsäuren vorliegend) und kein zusätzliches Wasser zusetzt. Wenn man höher konzentrierte Salpetersäure einsetzen will, ist es im allgemeinen erforderlich, eine entsprechende Menge Wasser zusätzlich einzuspeisen.

Die Temperatur, mit der Salpetersäure und gegebenenfalls das Wasser zugegeben wird, ist ohne besondere Bedeutung. Man kann beispielsweise Salpetersäure und gegebenenfalls Wasser mit Raumtemperatur einsetzen.

Für das erfindungsgemässe Verfahren ist es weiterhin wesentlich, dass das Nitriergemisch gut mit den Reaktionskomponenten vermischt wird. Hierzu können übliche Apparate verwendet werden, beispielsweise bei der Arbeitsweise im Rührkessel schnellaufende Rührer oder bei kontinuierlicher Arbeitsweise Schlaufenreaktoren. Die gute Vermischung des Nitriergemisches mit den Reaktionskomponenten ist notwendig, um lokale Überhitzungen des Reaktionsgemisches zu vermeiden.

Der Druck, bei dem das erfindungsgemässe Verfahren durchgeführt wird, ist ohne besondere Bedeutung. Vorzugsweise wird bei Normaldruck gearbeitet, man kann jedoch auch bei erhöhtem oder erniedrigtem Druck arbeiten. Man kann auch in Gegenwart eines Inertgases, z.B. Stickstoff, arbeiten.

Die Reaktionstemperatur beim erfindungsgemässen Verfahren ist im Bereich von 30 bis 60°C einzuhalten. Vorzugsweise arbeitet man im Bereich 35 bis 45°C. Um diese Temperaturen einhalten zu können, ist es erforderlich, Reaktionswärme, Verdünnungswärme und die Wärme aus der Abkühlung der Naphthalin-1,3,6-trisulfonsäure enthaltenden Schwefelsäurelösung in kurzer Zeit abzuführen. Die Abführung dieser Wärmen kann über die Behälterwandungen und/oder durch in das Reaktionsgemisch eintauchende Kühlschlangen erfolgen. Als besonders vorteilhaft hat sich ein externer Wärmeaustauscher erwiesen, durch den das Nitriergemisch während der Nitrierung im Kreislauf gepumpt und dort durch indirekte Kühlung mittels Kühlwasser abgekühlt wird.

Man kann das erfindungsgemässe Verfahren diskontinuierlich oder kontinuierlich betreiben. Bei diskontinuierlicher Fahrweise kann man beispielsweise in einem Rührwerksbehälter als Reaktor arbeiten. Bei kontinuierlicher Arbeitsweise kann man in mehreren Reaktoren, z.B. 2 oder 3 Reaktoren, arbeiten, die nacheinander kontinuierlich betrieben werden und wobei im ersten Reaktor die Nitrierung weitgehend durchgeführt wird, beispielsweise zu mindestens 80%, vorzugsweise zu mindestens 90%. Im zweiten Reaktor kann dann die Nitrierung vervollständigt werden. Gegebenenfalls kann auch noch ein dritter Reaktor vorhanden sein. Vorzugsweise wird das erfindungsgemässe Verfahren kontinuierlich durchgeführt. Dabei ist der erste Reaktor vorzugsweise ein Schlaufenreaktor, der zweite und gegebenenfalls dritte Reaktor vorzugsweise ein Rührkessel. Die im Schlaufenreaktor abzuführenden Wärmen werden vorzugsweise durch Umpumpen des Reaktorinhalts über einen im Kreislauf angeordneten Wärmeaustauscher abgeführt.

Bei einer bevorzugten Ausführungsform des erfindungsgemässen Verfahrens in diskontinuierlicher Arbeitsweise wird wie folgt verfahren: In einem Behälter wird soviel ausreagiertes Nitriergemisch vorgelegt, dass der Rührer des Behälters dieses gut rühren kann. Dann lässt man unter schnellem Rühren simultan über getrennte Stutzen z.B. 120 Mol-% 67 bis 68%ige Salpetersäure (sog. Azeotropsäure) mit einer Temperatur von 20 bis 25°C und 100 Mol-% gemäss F.I.A.T., Final Report, a.a.O. hergestellte, 140 bis 160°C heisse Lösung der Naphthalintrisulfonsäuren in ca. 100%iger Schwefelsäure so schnell zulaufen, dass im Reaktionsbehälter eine Temperatur von 35 bis 38°C eingehalten wird. Die Wärme aus der Reaktion, Verdünnung und Abkühlung des heissen Sulfieransatzes wird dabei durch indirekte Kühlung abgeleitet. Nach beendetem Zulauf lässt man noch 1 bis 2 Stunden bei 35 bis 38°C nachreagieren.

Bei einer bevorzugten Ausführungsform des erfindungsgemässen Verfahrens in kontinuierlicher Arbeitsweise, die auch bevorzugt technisch angewendet werden kann, wird wie folgt verfahren: Ein gemäss F.I.A.T., Final Report, a.a.O. erhaltenes Naphthalintrisulfonsäuregemisch, gelöst in ca. 100%iger Schwefelsäure, das ca. 33,8% Naphthalin-1,3,6-trisulfonsäure, ca. 5,8% Naphthalin-1,3,7-trisulfonsäure und ca. 3,7% Naphthalin-1,3,5-trisulfonsäure enthält, wird mit einer Temperatur von 150 bis 160°C zusammen mit wenig 100%iger Schwefelsäure in einen Schlaufenreaktor eindosiert, in dem ein zu ca. 95% ausreagiertes Nitriergemisch mit einer Kreiselpumpe schnell umgewälzt wird. Dabei wird die Naphthalintrisulfonsäuren enthaltende Schwefelsäurelösung so in den Schlaufenreaktor eingebracht, dass eine schnelle Vermischung und Abkühlung der heissen Sulfierschmelze mit der bzw. durch die Nitriermischung gewährleistet ist. Gleichzeitig wird in einem regelbaren Verhältnis zur Naphthalintrisulfonsäure enthaltenden Schwefelsäure ca. 68%ige Salpetersäure (Azeotropsäure) in den Schlaufenreaktor eingepumpt und intensiv mit dem Reaktorinhalt vermischt. Durch Kühlung des umgewälzten Reaktorinhalts mittels eines im Kreislauf angeordneten Wärmeaustauschers mit Kühlwasser wird eine Temperatur von 35 bis 38°C eingestellt. Nach einer mittleren Verweilzeit von ca. 20 bis 40 Minuten fliesst das zu ca. 95% ausreagierte Nitriergemisch über einen Überlauf zur Reaktion in einen Rührwerksbehälter. Dort kann die restliche Reaktionswärme durch indirekte Kühlung abgeführt werden. Nach einer weiteren mittleren Verweilzeit von ca. 10–50 Minuten fliesst die nahezu ausreagierte Nitriermischung in einen dritten Rührwerkskessel.

Das nach Durchführung des erfindungsgemässen Verfahrens vorliegende Gemisch kann beispielsweise wie in F.I.A.T., Final Report, a.a.O. beschrieben aufgearbeitet werden, beispielsweise durch Verdünnung mit Wasser, Austreiben der nitrosen Gase und Abtrennung der vorhandenen Schwefelsäure durch Zusatz von Calciumcarbonat als Calciumsulfat. Man kann aus dem dann vorliegenden, im wesentlichen 8-Nitronapthalin-1,3,6-trisulfonsäure enthaltenden Gemisch, ohne Zwischenisolierung dieser Säure (Nitro-T-Säure), diese direkt zu T-Säure umsetzen, indem man die vorliegende Lösung durch Reduktion nach Béchamps mit Eisen oder durch katalytische Hydrierung reduziert. Durch Versetzen der reduzierten Lösung mit Kochsalz und Ansäuern mit Salzsäure kann man T-Säure als saures Calcium-Natrium-Salz abscheiden und isolieren (s. hierzu z.B. F.I.A.T., Final Report, a.a.O.).

Nitro-T-Säure und T-Säure sind Zwischenprodukte zur Herstellung von H-Säure, die ihrerseits ein wichtiges Zwischenprodukt zur Herstellung von Farbstoffen ist (s. Ullmann, Enzyklopädie der technischen Chemie, 3. Auflage, 12. Band, Seite 621).

Die Nitrierausbeuten beim erfindungsgemässen Verfahren wurden wie folgt bestimmt: Die ausreagierte Nitriermischung wurde zunächst mit Wasser, dann zur Beseitigung gelöster nitroser Gase mit Harnstoff versetzt, anschliessend mit Natronlauge neutralisiert und dann mittels Hochdruckflüssigkeitschromatographie analysiert.

Wenn man reine, praktisch isomerenfreie Naphthalin-1,3,6-trisulfonsäure gelöst in Schwefelsäure einsetzt, so erhält man ca. 96% der Theorie an 8-Nitronaphthalin-1,3,6-trisulfonsäure (Nitro-T-Säure), ca. 3% der Theorie an 1-Nitronaphthalin-2,5,7-trisulfonsäure und ca. 0,5% der Theorie 2-Nitronaphthalin-3,6,8-trisulfonsäure, was einem Gesamtnitrierumsatz von 99,5% der Theorie entspricht.

Wenn man ein Naphthalin-1,3,6-trisulfonsäure enthaltendes Gemisch, gelöst in Schwefelsäure, einsetzt, wie es bei der Trisulfierung von Naphthalin anfällt, so erhält man beispielsweise beim Einsatz einer Naphthalintrisulfonsäurelösung in ca. 100%iger Schwefelsäure, die ca. 33,8% Naphthalin-1,3,6-trisulfonsäure, ca. 5,8% Naphthalin-1,3,7-trisulfonsäure und ca. 3,7% Naphthalin-1,3,5-trisulfonsäure enthält, ca. 96% der Theorie an Nitro-T-Säure, ca. 3% der Theorie an 1-Nitronaphthalin-2,5,7-trisulfonsäure und ca. 0,5% der Theorie an 2-Nitronaphthalin-3,6,8-trisulfonsäure als Nitrierprodukte der Naphthalin-1,3,6-trisulfonsäure. In diesem Fall erhält man weiterhin ca. 45% der Theorie 1-Nitronaphthalin-3,5,7-trisulfonsäure und ca. 7% der Theorie 2-Nitronaphthalin-3,5,7-trisulfonsäure als Nitrierprodukte der Naphthalin-1,3,7-trisulfonsäure, von der ca. 31% der Theorie unnitriert bleiben. Weiterhin erhält man ca. 62% der Theorie 1-Nitronaphthalin-4,6,8-trisulfonsäure und ca. 8% der Theorie 2-Nitronaphthalin-4,6,8-trisulfonsäure als Nitrierprodukte der Naphthalin-1,3,5-trisulfonsäure, von der ca. 19% der Theorie unnitriert bleiben.

Das erfindungsgemässe Verfahren besitzt eine Reihe von Vorteilen gegenüber den aus der Literatur bekannten Verfahren. So kann die Nitrierreaktion in wesentlich kürzeren Reaktionszeiten als bisher durchgeführt werden. Weiterhin können Raum/Zeit-Ausbeuten von etwa 0,7 bis 1,5 Mol Nitro-T-Säure pro Liter Behältervolumen und Stunde erreicht werden, was ein Vielfaches der aus der Literatur bekannten Raum/Zeit-Ausbeute ist. Wegen der ausserordentlich grossen Raum/Zeit-Ausbeuten können die Reaktorvolumina klein gehalten werden, womit sich nur ein geringer Vorrat an thermolabilem, noch nicht verdünntem Nitriergemisch ergibt. Durch die direkte Vermischung der Naphthalin-1,3,6-trisulfonsäure enthaltenden Schwefelsäurelösung mit dem Nitriergemisch und durch die schnelle Nitrierreaktion wird ein Auskristallisieren von Naphthalintrisulfonsäuren vermieden. Wenn man die direkt bei der Trisulfierung von Naphthalin anfallende Lösung von Naphthalintrisulfonsäuren in Schwefelsäure einsetzt, erübrigt sich eine Abkühlung dieser Schwefelsäurelösung, die bisher für notwendig erachtet wurde. Die Reaktionsprodukte sind reiner als bei den bisherigen Verfahren, insbesondere treten weniger Nebenprodukte auf, welche die katalytische Hydrierung der Nitro-T-Säure zu T-Säure verzögern oder blockieren können. Im Gegensatz zu den bekannten Verfahren, bei denen eine 86%ige Salpetersäure verwendet wird, die 12% Schwefelsäure und 2% Wasser enthält, kann die preiswerte 67- bis 68%ige Salpetersäure (sog. Azeotropsäure) eingesetzt werden. Schliesslich sind die mit dem erfindungsgemässen Verfahren erzielbaren Ausbeuten an Nitro-T-Säure bezogen auf Naphthalin-1,3,6-trisulfonsäure als optimal anzusehen, da bei praktisch vollständiger Nitrierung der Naphthalin-1,3,6-trisulfonsäure nur 3 bis 4% unvermeidbare isomere Nitro-Naphthalin-trisulfonsäuren entstehen.

Obwohl das Nitriergemisch eine übersättigte Lösung von Nitro-T-Säure in ca. 90%iger Schwefelsäure ist und zur Kristallisation neigt, werden überraschenderweise keine Störungen bei der kontinuierlichen Durchführung des erfindungsgemässen Verfahrens beobachtet.

Beispiel 1

In einem 2 l Mehrhalskolben, der mit Thermometer, Rührer, Rückflusskühler und 2 Tropftrichtern ausgerüstet ist, legt man bei 30°C 1140 g zu ca. 95 bis 99% ausreagiertes Nitriergemisch, welches entsprechend F.I.A.T., Final Report, Nr. 1016, Seiten 32 bis 39, durch Nitrierung der Naphthalintrisulfonsäuremischung aus der Naphthalintrisulfierung mit Mischsäure (86% Salpetersäure, 12% Schwefelsäure, 2% Wasser) hergestellt wurde, vor und lässt im Verlaufe von ca. 40 Minuten unter starkem Rühren simultan aus verschiedenen Tropftrichtern 1430 g 150 bis 160°C heisse Sulfierschmelze (Reaktionsmischung aus der Naphthalintrisulfierung), die 33,8 Gew.-% Naphthalin-1,3,6-trisulfonsäure, 5,8 Gew.-% Naphthalin-1,3,7-trisulfonsäure und 3,7 Gew.-% Naphthalin-1,3,5-trisulfonsäure neben wenig Naphthalin-1,3,5,7-tetrasulfonsäure und sonstigen Sulfier- und Sulfoxidationsprodukten enthält und 198 g 67- bis 68%ige Salpetersäure (Azeotropsäure) zutropfen. Dabei wird eine Reaktionstemperatur von 36°C durch indirekte Kühlung des Reaktionskolbens mit Eiswasser eingehalten. Nach beendetem Zulaufen rührt man noch 60 Minuten bei 36°C nach und hydrolysiert dann den Ansatz, indem man diesen vorsichtig in einen Kolben laufen lässt und dabei gleichzeitig 1160 g Wasser zudosiert. Während dieser Operation wird die Mischung ca. 120°C heiss und nitrose Gase entweichen. Die Reaktionsmischung wird nach beendeter Verdünnung auf 60 bis 70°C abgekühlt.

Eine Probe wird zur Entfernung gelöster nitroser Gase mit Harnstoff oder Amidosulfonsäure versetzt, mit 50%iger Natronlauge neutralisiert und dann mittels Hochdruckflüssigkeitschromatographie analysiert.

Als Hauptbestandteile der verdünnten Nitriermischung wurden gefunden:

22,5 Gew.-% Nitro-T-Säure

2,7 Gew.-% 1-Nitronaphthalin-3,5,7-trisulfonsäure

1,4 Gew.-% 1-Nitronaphthalin-4,6,8-trisulfonsäure

0,7 Gew.-% 1-Nitronaphthalin-2,5,7-trisulfonsäure

0,3 Gew.-% 2-Nitronaphthalin-3,5,7-trisulfonsäure.

Die Ausbeute an Nitro-T-Säure betrug 96% der Theorie, bezogen auf eingesetzte Naphthalin-1,3,6-trisulfonsäure.

Beispiel 2

In einem Schlaufenreaktor mit einem Leervolumen von 2 m³, dosiert man kontinuierlich in zu ca. 95 bis 99% ausnitrierte Produktmischung, die im Kreislauf gepumpt wird, 4668 kg/h ca. 150 bis 160°C heisse Sulfierschmelze und gleichzeitig 645 kg/h 67,5%ige Salpetersäure (Azeotropsäure) ein, wobei im Schleifenreaktor eine Reaktionstemperatur von 35 bis 40°C eingehalten wird. Die Sulfierschmelze wurde erhalten, indem Naphthalin gemäss F.I.A.T., Final Report, Nr. 1016, Seiten 32 bis 39, trisulfiert wurde.

Das zu ca. 95 bis 99% ausnitrierte Reaktionsgemisch läuft dann über einen Überlauf zur Nachreaktion bei 35–40°C in einen Rührwerksbehälter und von dort nach einer mittleren Verweilzeit von 20 bis 25 Minuten in einen weiteren Rührwerksbehälter.

Die Ausbeute an Nitro-T-Säure betrug 97,5% der Theorie, bezogen auf eingesetzte Naphthalin-1,3,6-trisulfonsäure (berechnet aus der bei der Weiterverarbeitung der Nitro-T-Säure erzielten Ausbeute an H-Säure).

**Patentansprüche**

1. Verfahren zur Herstellung von 8-Nitronaphthalin-1,3,6-trisulfonsäure aus Naphthalin-1,3,6-trisulfonsäure oder diese enthaltenden Gemischen durch Nitrierung mit Salpetersäure in Schwefelsäure, dadurch gekennzeichnet, dass man zu mindestens 80% ausreagiertes Nitriergemisch vorlegt, Naphthalin-1,3,6-trisulfonsäure oder diese enthaltende Gemische gelöst in Schwefelsäure zufügt, gleichzeitig 1,0 bis 1,4 Mol Salpetersäure pro Mol Naphthalin (in Form von Naphthalinsulfonsäure vorliegend) und gegebenenfalls Wasser in der Weise zufügt, dass man eine Schwefelsäurekonzentration im Bereich 86 bis 94 Gew.-% (Schwefelsäure bezogen auf Summe aus allem vorhandenen Wasser und Schwefelsäure) während der Nitrierung einhält, das Reaktionsgemisch gut durchmischt und die Reaktionstemperatur im Bereich 30 bis 60°C hält.

2. Verfahren nach Anspruch 1, dadurch gekennzeichnet, dass man bei diskontinuierlicher Durchführung 5 bis 5.000 Gew.-% Naphthalin-1,3,6-trisulfonsäure enthaltende Schwefelsäurelösung, bezogen auf vorgelegtes ausreagiertes Nitriergemisch, einsetzt.

3. Verfahren nach Anspruch 1, dadurch gekennzeichnet, dass man bei kontinuierlicher Durchführung maximal soviel Naphthalin-1,3,6-trisulfonsäure enthaltende Schwefelsäurelösung eingibt, dass die eingegebene Menge Naphthalin-1,3,6-trisulfonsäure im ersten Durchgang zu mindestens 80% nitriert wird.

4. Verfahren nach Ansprüchen 1 bis 3, dadurch gekennzeichnet, dass man 1,1 bis 1,25 Mol Salpetersäure pro Mol Naphthalin (in Form von Naphthalinsulfonsäuren vorliegend) einsetzt.

5. Verfahren nach Ansprüchen 1 bis 4, dadurch gekennzeichnet, dass man 60- bis 100%ige Salpetersäure einsetzt.

6. Verfahren nach Ansprüchen 1 bis 5, dadurch gekennzeichnet, dass man 67- bis 68%ige Salpetersäure einsetzt.

7. Verfahren nach Ansprüchen 1 bis 6, dadurch gekennzeichnet, dass man die Schwefelsäurekonzentration im Bereich 88 bis 92% während der Nitrierung einhält.

8. Verfahren nach Ansprüchen 1 bis 7, dadurch gekennzeichnet, dass man die Reaktionstemperatur im Bereich 35 bis 45°C hält.

9. Verfahren nach Ansprüchen 1, 2 und 4 bis 8, dadurch gekennzeichnet, dass man bei diskontinuierlicher Fahrweise in einem Rührwerksbehälter arbeitet.

10. Verfahren nach Ansprüchen 1 und 3 bis 8, dadurch gekennzeichnet, dass man bei kontinuierlicher Arbeitsweise in mehreren Reaktoren arbeitet, wovon der erste Reaktor ein Schlaufenreaktor ist, in dem die Nitrierung zu mindestens 80% durchgeführt wird.

## Claims

1. Process for the preparation of 8-nitronaphthalene-1,3,6-trisulphonic acid from naphthalene-1,3,6-trisulphonic acid or mixtures containing the latter by nitration with nitric acid in sulphuric acid, characterised in that a nitration mixture which has reacted to the extent of at least 80% is initially introduced, naphthalene-1,3,6-trisulphonic acid or mixtures containing the latter, dissolved in sulphuric acid, are added, at the same time 1.0 to 1.4 mols of nitric acid per mol of naphthalene (present in the form of naphthalenesulphonic acid) and optionally water are added in such a way that a sulphuric acid concentration in the range of 86 to 94% by weight (of sulphuric acid, relative to the sum of all the water present, and sulphuric acid) is maintained during the nitration, the reaction mixture is mixed thoroughly and the reaction temperature is maintained in the range from 30 to 60°C.

2. Process according to Claim 1, characterised in that, in discontinuous operation, 5 to 5,000% by weight, relative to reacted nitration mixture initially introduced, of sulphuric acid solution containing naphthalene-1,3,6-trisulphonic acid are employed.

3. Process according to Claim 1, characterised in that, in continuous operation, the amount of sulphuric acid solution, containing naphthalene-1,3,6-trisulphonic acid, introduced is at most such that the amount of naphthalene-1,3,6-trisulphonic acid introduced is nitrated to the extent of at least 80% in the first pass.

4. Process according to Claims 1 to 3, characterised in that 1.1 to 1.25 mols of nitric acid are employed per mol of naphthalene (present in the form of naphthalene-sulphonic acids).

5. Process according to Claims 1 to 4, characterised in that 60 to 100% strength nitric acid is employed.

6. Process according to Claims 1 to 5, characterised in thet 67 to 68% strength nitric acid is employed.

7. Process according to Claims 1 to 6, characterised in that the sulphuric acid concentration is maintained in the range from 88 to 92% during the nitration.

8. Process according to Claims 1 to 7, characterised in that the reaction temperature is kept in the range from 35 to 45°C.

9. Process according to Claims 1, 2 and 4 and 8, characterised in that, in discontinuous operation, the reaction is carried out in a stirred vessel.

10. Process according to Claims 1 and 3 to 8, characterised in that, in continuous operation, the reaction is carried out in a plurality of reactors, of which the first reactor is a loop reactor in which the nitration is carried out to the extent of at least 80%.

## Revendications

1. Procédé de production d'acide 8-nitronaphtalène-1,3,6-trisulfonique à partir d'acide naphtalène-1,3,6-trisulfonique ou de mélanges contenant cet acide, par nitration avec de l'acide nitrique dans l'acide sulfurique, caractérisé en ce qu'on introduit tout d'abord du mélange nitré ayant réagi à 80% au moins, on ajoute de l'acide naphtalène-1,3,6-trisulfonique ou des mélanges contenant cet acide dissous dans de l'acide sulfurique, on ajoute en même temps 1,0 à 1,4 moles d'acide nitrique par mole de naphtalène (présent sous la forme d'acide naphtalènesulfonique) et le cas échéant de l'eau de manière qu'on maintient pendant la nitration une concentration en acide sulfurique dans la plage de 86 à 94 % en poids (d'acide sulfurique par rapport à la somme de toute l'eau présente et de l'acide sulfurique), on agite correctement le mélange réactionnel et on maintient la température de réaction dans la plage de 30 à 60°C.

2. Procédé suivant la revendication 1, caractérisé en ce qu'on utilise, dans le cas d'une mise en œuvre discontinue, 5 à 5000% en poids de solution d'acide sulfurique contenant de l'acide naphtalène-1,3,6-trisulfonique, par rapport au mélange nitré ayant réagi, introduit au préalable.

3. Procédé suivant la revendication 1, caractérisé en ce qu'on introduit au maximum, dans le cas d'une conduite continue, une quantité suffisante de solution d'acide sulfurique contenant de l'acide naphtalène-1,3,6-trisulfonique pour que la quantité introduite d'acide naphtalène-1,3,6-trisulfonique soit nitrée au moins à 80% lors du premier passage.

4. Procédé suivant les revendications 1 à 3, caractérisé en ce qu'on utilise 1,1 à 1,25 mole d'acide nitrique par mole de naphtalène (présent sous la forme d'acides naphtalènesulfoniques).

5. Procédé suivant les revendications 1 à 4, caractérisé en ce qu'on utilise de l'acide nitrique à 60–100%.

6. Procédé suivant les revendications 1 à 5, caractérisé en ce qu'on utilise de l'acide nitrique à 67–68%.

7. Procédé suivant les revendications 1 à 6, caractérisé en ce qu'on maintient la concentration en acide sulfurique dans la plage de 88 à 92% pendant la nitration.

8. Procédé suivant les revendications 1 à 7, caractérisé en ce qu'on maintient la température de réaction dans la plage de 35 à 45°C.

9. Procédé suivant les revendications 1, 2 et 4 à 8, caractérisé en ce qu'on opère dans un récipient équipé d'un agitateur lors de la mise en œuvre discontinue.

10. Procédé suivant les revendications 1 et 3 à 8, caractérisé en ce qu'on opère lors de la mise en œuvre continue dans plusieurs réacteurs dont le premier est une colonne à bulles à écoulement en boucle dans laquelle la nitration est effectuée au moins à 80%.